# EUROPEAN PATENT SPECIFICATION

(11) **EP 3 539 537 B1**
(45) Date of publication and mention of the grant of the patent: **15.07.2026**
(21) Application number: 17840578.3
(22) Date of filing: 09.11.2017
(51) Int. Cl.: A61K 9/20, A61K 31/4418, A61P 1/16

(54) **PHARMACEUTICAL USE OF AN EXTENDED-RELEASE COMPOSITION CONTAINING PIRFENIDONE FOR THE TREATMENT AND REVERSAL OF HUMAN STEATOHEPATITIS (NAFLD/NASH)**
PHARMAZEUTISCHE VERWENDUNG EINER RETARD-ZUSAMMENSETZUNG MIT PIRFENIDON ZUR BEHANDLUNG UND UMKEHRUNG VON MENSCHLICHER STEATOHEPATITIS (NAFLD/NASH)
UTILISATION PHARMACEUTIQUE D'UNE COMPOSITION À BASE DE PIRFÉNIDONE À LIBÉRATION PROLONGÉE (PFD-LP) POUR LA RÉVERSION DE LA STÉATOSE HÉPATIQUE HUMAINE (NAFLD/NASH)

(30) Priority: 11.11.2016 MX 2016014775
(43) Date of publication of application: 18.09.2019
(73) Proprietor: EXCALIBUR PHARMACEUTICALS, INC., New York, NY 10016 (US)
(72) Inventor: ARMENDÁRIZ BORUNDA, Juan Socorro, Ciudad De México 04899 (MX); MAGAÑA CASTRO, José Agustín Rogelio, Ciudad De México 04899 (MX); HERNÁNDEZ ALDANA, Nadiel, Ciudad De México 04899 (MX)
(74) Representative: Jacobacci & Partners France
(86) International application number: PCT/MX2017/000129
(87) International publication number: WO 2018/088886

(56) References cited:
- EP-A1- 1 356 816
- EP-A1- 2 907 506
- WO-A1-2010/054294
- WO-A2-2005/000227
- JOSE MACIAS-BARRAGAN ET AL: "1541 Pirfenidone LP activates PPARalpha and LXRalpha and results in decreased expression of proinflammatory cytokines and improvement of NASH features induced by high fat/carbohydrate diet", HEPATOLOGY - SPECIAL ISSUE: THE 67TH ANNUAL MEETING OF THE AMERICAN ASSOCIATION FOR THE STUDY OF LIVER DISEASES: THE LIVER MEETING 2016, vol. 64, no. S1, October 2016 (2016-10-01), pages 767A - 768A, XP002779382
- NAKANISHI H ET AL: "Pirfenidone inhibits the induction of iNOS stimulated by interleukin-1beta at a step of NF-kappaB DNA binding in hepatocytes", JOURNAL OF HEPATOLOGY, ELSEVIER, AMSTERDAM, NL, vol. 41, no. 5, 1 November 2004 (2004-11-01), pages 730 - 736, XP004617750, ISSN: 0168-8278, DOI: 10.1016/J.JHEP.2004.07.007
- MACIAS-BARRAGAN J ET AL: "P428 5-METHYL-1-PHENYL-2-(1H)-PYRIDONE TREATMENT IMPROVES MARKERS OF HEPATIC FUNCTION AND FIBROSIS IN STEATOSIS INDUCED BY HIGH FAT/CARBOHYDRATE DIET", JOURNAL OF HEPATOLOGY; ABSTRACTS OF THE INTERNATIONAL LIVER CONGRESS(TM) 2014 - 49TH ANNUAL MEETING OF THE EUROPEAN ASSOCIATION FOR THE STUDY OF THE LIVER, ELSEVIER, AMSTERDAM, NL; LONDON, UNITED KINGDOM, vol. 60, no. 1, Suppl, 1 January 2014 (2014-01-01), pages S210, XP028641723, ISSN: 0168-8278, [retrieved on 20140424], DOI: 10.1016/S0168-8278(14)60590-0
- OZES O N ET AL: "697 Preclinical activity of pirfenidone (5-methyl-1phenyl-2(IH)-pyridone) in cell-based models of nonalcoholic steatohepatitis", HEPATOLOGY, JOHN WILEY & SONS, INC, US, vol. 34, no. 4, 1 January 2003 (2003-01-01), pages 495A, XP004623912, ISSN: 0270-9139, DOI: 10.1016/S0270-9139(03)80739-5
- DATABASE WPI Section Ch Week 200629, Derwent World Patents Index; Class B03, AN 2006-273778, XP002779350, WU J: "Use of pirfenidone for treating hepatic injury and necrosis and acute lung injury"
- DATABASE WPI Section Ch Week 201139, Derwent World Patents Index; Class A96, AN 2011-D92901, XP002779383, LI X: "Sustained-release tablet comprises pirfenidone, substance capable of releasing active ingredient, and additive"
- DATABASE WPI Section Ch Week 201427, Derwent World Patents Index; Class A96, AN 2014-F77081, XP002779351, DENG C; LANG T; SHI W: "Pharmaceutical composition used for treating hepatic fibrosis, liver fibrosis, liver cirrhosis, and liver cancer comprises pirfenidone, inosine, and auxiliary materials"

## Description

### FIELD OF INVENTION

The present invention is related to a pharmaceutical composition in the form of an extended-release tablet that contains pirfenidone (PFD-LP) that is able to induce reduction/elimination of hepatic lipid accumulations or steatosis, and its therapeutic application in reversal of nonalcoholic steatohepatitis (NASH).

### BACKGROUND

### Cirrhosis

Cirrhosis is the final stage of advanced liver fibrosis (ALF), which is defined as an excessive accumulation of extracellular matrix (ECM), or interstitial scar, that is consequence of a chronic liver injury.

Due to the regenerative capacity of the liver, cirrhosis is a pathological process which can develop slowly. In addition, is known that it is a process in which genetic factors are also involved.

Changes in medical treatment against advanced liver fibrosis continue in progress, leading the implementation of antifibrogenic and preventive therapies that currently seem to offer hope to millions of patients worldwide.

ALF, characterized by an increase in the synthesis of ECM as well as a change in its composition, leads to an abnormal remodeling of the liver tissue and consequent distortion of the organ's architecture. This leads to a decrease in liver function.

### Physiopathology

The physiopathology of this disease is given by an inflammatory process, in which there is an increase in the expression of proinflammatory cytokines such as Interleukin-6 (IL-6), inteleukin-1β (IL-1β) and Tumor Necrosis Factor-alpha (TNF-α). The inflammation is accompanied by oxidative stress caused by the decrease in the expression of antioxidant enzymes such as superoxide dismutase (SOD) and catalase (CAT), and an increase in the production of free radicals such as superoxide anion, hydroxyl anion, and hydrogen peroxide, among others. Likewise, there is a fibrogenic process mediated, above all, by the Transforming Growth Factor beta 1 (TGF-β1) that induces an increase in the expression of type 1 collagen (COL-1), the main component of the ECM (Extracellular matrix), also the growth factors that favor the proliferation of Hepatic Stellate Cells are increased, and in the Tissue Inhibitor of Metalloprotease 1 (TIMP-1). In addition to the above-mentioned information, there is a decrease in the expression of ECM metalloproteases. All this leads to the imbalance between the synthesis and degradation of the ECM, which favors its accumulation.

### Production of Extracellular Matrix

The definition of the cellular components of the ECM has allowed advances in the field of medical therapy against the disease. Hepatic stellate cells (HSC) are the main source of ECM in cirrhosis. These cells are found in the space of Disse, a site between hepatic sinusoids and hepatocytes. The HSC is the main site where vitamin A is stored in the form of retinoids, and this vitamin is the one that will characterize HSCs in a quiescent phenotype.

However, HSCs can migrate through chemoattractant cytokines (chemokines). Among the various molecules that can act as chemokines are PDGF, MCP-1 and CXCR3. Recently, the mechanism by which the PDGF participates in this process has been described. It is said that it does so by diffusion in the membrane of other cells, causing them to move by dragging to other sites.

### Fibrogenesis

HSCs generate fibrosis not only by the increase in ECM production, but also by the proliferation of ECM producing cells. The main studied component of the hepatic scar is collagen type 1 (COL-1). Whose expression does an increasing number of stimuli and pathways regulate both transcriptionally and post-transcriptionally. The most potent stimulus is that observed by TGF-β1, which is given by both paracrine and autocrine agents. Signaling downstream in the TGF-β1 cascade includes the family of bifunctional molecules known as Smads. TGF-β1 also stimulates the production of other components of ECM such as fibronectin and proteoglycans. The stimulation of TGF-β1 to HSC is mediated by peroxidation of hydrogen and dependent mechanisms of C / EBPβ.

Lipid peroxidation are important products that will also generate a stimulus in the production of ECM. The effect is enhanced by a loss of the antioxidant capacity of the HSC.

The connective tissue growth factor (CTGF/CCN2) is also considered as an important stimulus in the HSC for the production of ECM. It may also be activated by periods of hyperglycemia and hyperinsulinemia. The stimulation of CTGF has been considered dependent on TGF-β1.

### Contractility

The contractility of HSC could be the main determinant of the early or late increase in portal resistance in cirrhosis. The collagen bands prevent a normal flow of blood in the portal pathway due to a constriction of the sinusoids and a contractility throughout the cirrhotic liver.

Endothelin-1 and nitric oxide are the main regulators of contractility in HSC. Also included in this list are angiotensinogen II, eicosanoids, atrial natriuretic peptide, somatostatin, carbon monoxide, among many others. The expression of α-SMA protein of cytoskeleton is increased; this confers a potential contractility to the HSC.

### DIRECT BACKGROUND OF INVENTION

The main public health problems in developed countries are overweight and obesity [1]. Therefore, pathologies such as nonalcoholic fatty liver disease/nonalcoholic steatohepatitis (hereinafter identified as NAFLD/NASH) associated with the metabolic syndrome; continue to increase their prevalence throughout the world. Non-alcoholic fatty liver is predicted to be a major cause of liver-related morbidity and mortality over the coming decades.

The pathogenesis of fatty liver by alcohol and the one induced by causes beyond the consumption of alcohol such as non-alcoholic steatohepatitis is based on the accumulation of triglycerides that leads to hepatic steatosis. After storage of aberrant chronic lipids, a second damage is generated by inflammation, orchestrated by an inflammatory reaction and production and infiltration of cells and cytokines [2]. The most severe histological form of nonalcoholic fatty liver is non-alcoholic steatohepatitis (NASH), characterized by infiltration of inflammatory cells and liver damage, in the presence or absence of cirrhosis.

It is estimated that 20-30% of people in Western countries suffer from non-alcoholic fatty liver; the propensity to develop this condition seems to be related to racial-ethnic origin, which could be represented by 45% Hispanics, 33% Caucasians and 24% African-Americans. It has been observed that in the presence of normoglycemia and normal or moderate body weight, NAFLD is characterized by laboratory levels and clinical data similar to those observed in diabetes and obesity [1, 3].

Recent studies show that increasing the amount of dietary cholesterol results in more severe inflammation, hepatocellular injury and fibrosis [4]. On the other hand, high-carbohydrate diets have been linked to an increase in the level of insulin that contributes to elevated triglyceride levels [5]. Fructose and sucrose have a particular important role in the development of metabolic disorders. In addition, studies have shown that high-fructose diet is associated with the development of liver inflammation, NASH and established fibrosis [6]. In response to chronic excess of calories, an upregulation of the main participant IL-17 of the pro-inflammatory axis has been observed. Such an event is linked to liver damage due to the release of mediators of Kupffer cells and hepatic stellate cells (HSC) and not by hepatocytes, endothelial cells or neutrophils [7].

With regard to obesity, adipogenesis is regulated in a complex way by hormones, lipids, sugar metabolites and adipokines, as well as by other inflammatory cytokines. Taken together, these factors exacerbate the onset of NASH, which promotes the secretion of several proinflammatory cytokines such as TGF-β1, IL-1β, IL-16, which lead to inflammation of the liver [8]. In addition, it has been observed that IL-17A stimulates the production of C-reactive protein (CRP) by hepatocytes [9]. Recent studies have shown that the combination of immunoregulatory cytokines such as TGF-β1 and IL-6 inhibits the generation of regulatory T cells (Treg) that cause the loss of immune tolerance that trigger an increased production of Th17 cells and consequently higher levels of IL-17A that generate a kind of proinflammatory feedback response [10, 11, 12].

The transcription factors that regulate fatty acid oxidation and synthesis have been implicated in the development of nonalcoholic fatty liver, as well as in anti-inflammatory processes [13, 14]. In this sense, nuclear receptors PPARalfa, PPARgamma and LXR represent the main target of therapeutic strategies aimed at the improvement of hepatic steatosis and inflammation. PPARalpha regulates the oxidation of fatty acids by increasing the expression of genes such as CPT1A, which in turn internalizes lipids to the mitochondria to obtain energy from them [15]. On the other hand, LXR has long been known for its ability to increase the rate of lipogenesis by promoting the expression of another master transcriptional factor such as SREBP1 [16].

Finally, it has been shown that the activation of LXR alpha inhibits the expression of genes that code for mediators of inflammation [14, 16].

### Pirfenidone

Pirfenidone is a medicine made up of a small molecule, whose chemical name is 5-methyl-1-phenyl-2-(1H)pyridone. It is a non-peptide synthetic molecule with a molecular weight of 185.23 Daltons. Its chemical formula is C₁₂H₁₁NO, and its structure is known. At present, Pirfenidone is under clinical evaluation as a broad-spectrum anti-fibrotic drug. Pirfenidone has anti-fibrotic and anti-inflammatory properties that are reflected in its activity of reducing the expression of TGF-β1, TNF-α, PDGF and most importantly, the expression of different types of collagen. Currently, clinical studies are being conducted in humans with respect to pulmonary fibrosis, chronic renal failure secondary to renal glomerulosclerosis, liver cirrhosis and capsular breast contracture. There are basic and clinical research works, already published or in the process of being published, which have shown that pirfenidone reduces the progressive progress of lesions with fibrosis. Most importantly, pirfenidone carries out its cellular and molecular functions in a safe and non-toxic manner. On the other hand, it is known that pirfenidone prevents the formation of fibrotic lesions after damage to a certain organ, for example liver, skin, kidney, etc.

It is known that one of the mechanisms by which Pirfenidone performs its therapeutic effects is through the modulation of the action of several cytokines. Pirfenidone is a potent inhibitor of fibrogenic cytokines and TNF-α.

Document WO2005/000227 describes the use of pirfenidone in the treatment of non-alcoholic steatohepatitis (NASH).

### OBJECTS OF INVENTION

**New molecular mechanisms of action of extended-released Pirfenidone (PFD-LP) have been discovered, showing that the compound is active in reducing the deleterious effects of non-alcoholic steatohepatitis (NASH), which is the object of this invention.**

The object of the present invention is a pharmaceutical composition in the form of an extended-release tablet comprising 100 mg, 200 mg, 300 mg, 400 mg or 600 mg of Pirfenidone, for use in the treatment of non-alcoholic steatohepatitis (NASH).

The use of the pharmaceutical composition in the form of an extended-release tablet may be helpful for the regression of advanced hepatic fibrosis.

The use of the pharmaceutical composition in the form of an extended-release tablet may be helpful for the regression of NASH, which occurs through the decrease of serum cholesterol and triglycerides.

The use of the pharmaceutical composition in the form of an extended-release tablet may be helpful for the regression and treatment of NASH, which occurs by decreasing the content of the hepatic fat accumulation, both in the form of macrosteatosis and microsteatosis.

The present invention is illustrated, but not limited by, the aforementioned object; for the treatment or regression of non-alcoholic steatohepatitis (NASH), demonstrating that the pharmaceutical composition in the form of extended-release tablets containing pirfenidone favors the diminution of the hepatotoxic effect of pirfenidone, previously disclosed in the current state of the art.

### BRIEF DESCRIPTION OF THE FIGURES

Other features and advantages of the invention will be clear from the following detailed description of the objectives and preferred embodiments of the appended claims and of the accompanying figures (only the treatment of NASH is according to the invention), wherein:
**Fig. 1****(A-D):** Shows the body weights, the glucose levels and the levels of the ALT and AST liver enzymes of the mice.
**Fig. 2****:** Histological examination of the tissues of the livers of the mice included in the control, HF and those treated with PFD-LP.
**Fig. 3****(A-F):** Shows the analysis of proinflammatory cytokines in the serum of the mice included in each of the indicated groups.
**Fig. 4****:** Shows the expression of profibrogenic and proinflammatory marker genes.
**Fig. 5****:** Shows the western blots with the protein expression of the mediators of fat metabolism in NAFLD/NASH, the key metabolic transcription factors LXR and PPARalpha were evaluated in the liver tissue.
**Fig. 6****:** Shows the western blots with the protein expression of the key transcriptional factors in the regulation of the inflammatory process in the liver, such as PPARgamma and NFkB.

### DETAILED DESCRIPTION OF THE INVENTION (only the treatment of NASH is according to the invention)

### ANIMALS USED IN THE EXPERIMENTS

Mice of six to eight weeks of age, male C57BL/6NHsd (Harlan, Mexico City) were housed in an atmosphere of 22 ± 2 ° C in 12-hour light/dark cycles. 5-7 mice were randomly assigned to the standard Chow diet (control) or high-fat/high carbohydrate (HF) diet for 16 weeks. The control group received diet Harlan TM-2018 (18% of calories from fat) and had free access to pure water, while the HF group received Harlan diet TD-06414 (60% of calories from lipids) and had free access to water with high fructose enriched at a concentration of 42 g/L (proportions in 55% fructose and 45% sucrose). The group of PFD mice (HF + PFD) received the HF diet for 8 weeks, followed by the HF diet for 8 weeks and 100 mg/kg/day of PFD extended release formulation. All regimens received 0.1 ml of vehicle. After a night of fasting, blood samples were analyzed. The weights of the mice and glucose were recorded weekly from start to sacrifice.

BODY WEIGHT, GLUCEMIA, CHOLESTEROL, TRIGLYCERIDES, VLDL CHOLESTEROL AND AMINOTRANSFERASES.

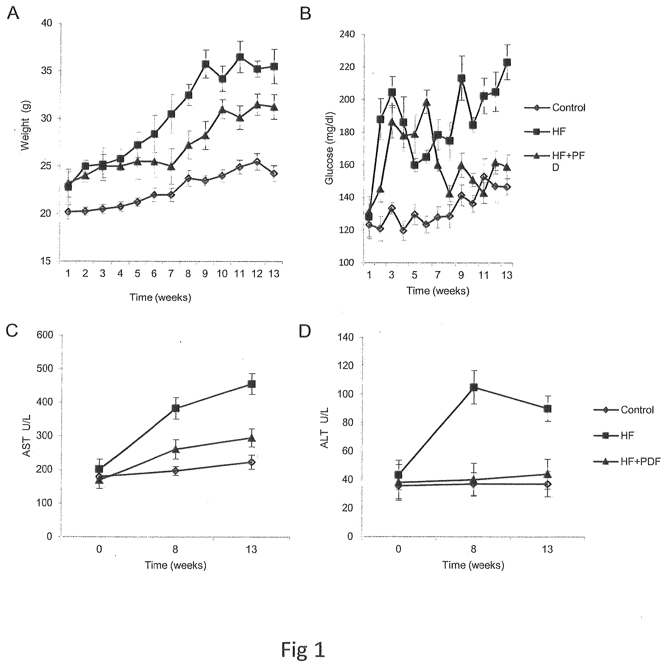

The characteristics of mice with steatohepatitis (NASH/NAFLD) induced by high-fat diet (FH) are shown in Fig. 1A-1D. Significant differences were found between the groups, being the mice of the HF group those that gained the most weight (up to 37% against the control) and the HF + PFD-LP group having 11% less weight than the HF group (Fig. 1A ). Regarding glycemia, higher serum glucose levels were observed in the HF group compared to the control group, with a significant difference from week 9 to 13. However, in the last five weeks of treatment, there was no significant difference between HF + PFD-LP vs. control group (Fig. 1B). We also found higher serum levels of AST (Fig. 1C), ALT (Fig. 1D), and cholesterol, triglycerides and VLDL-cholesterol (Table 1) in the HF group compared to HF + PFD or control group.

**Table 1. Determinations in serum of cholesterol, triglycerides and VLDL-cholesterol**

| | Control | HF | HF+PFD |
|---|---|---|---|
| Cholesterol (mg/dl) | 78 ± 22 (10) | 153 ± 27 (10)^{a} | 120 ± 24 (8) |
| Triglycerides (mg/dl) | 128 ± 28 (10) | 212 ± 44 (10)^{a} | 127 ± 38 (8)^{b} |
| VLDL (mg/dl) | 30 ± 6 (10) | 34 ± 7 (10) | 25 ± 8 (8) |

The values are the averages ± SD. The numbers of the mice are
indicated in parentheses.
^{a} P < 0.05 compared with the control.
^{b} *P* < 0.05 compared with HF.

### HISTOLOGY

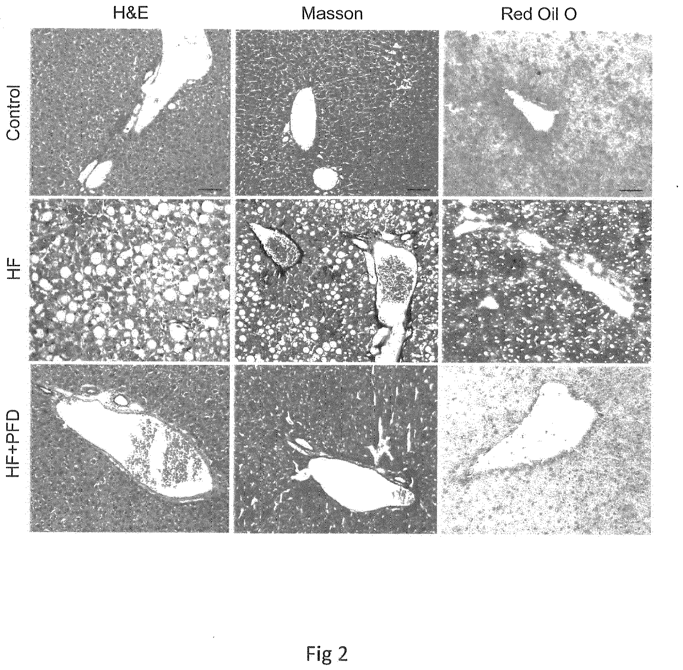

Histological examination of liver tissues of the HF group showed substantial microvesicular steatosis and macrovesicular steatosis with inflammatory changes (Fig. 2). Steatosis in the HF group was predominantly macrovesicular and was severe in acinar zone 1 with severe microvesicular steatosis and macrovesicular steatosis in acinar zone 2. Evidence of severe microvesicular steatosis was found in acinar zone 3 and moderate balloon degeneration in hepatocytes in that area; the inflammation was predominantly periportal with neutrophils and mononuclear cells, which also shows periportal fibrosis with a score of 1 (0-4), such histological changes are compatible with stage 1C of hepatic fibrosis and NAFLD in class 3. PFD-LP induced a dramatic decrease in liver fat content. These results correlate with gross hepatic observations. Regarding central and peripheral fat, HF + PFD-LP showed a lower level of fat compared to the HF group.

### ANALYSIS OF CYTOKINES IN SERUM

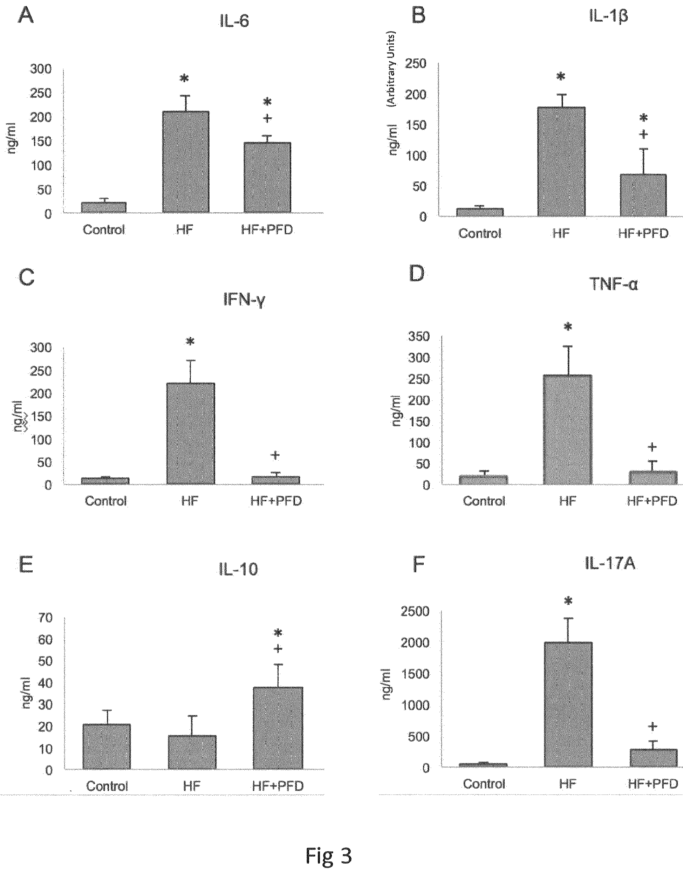

In order to correlate the histological results with systemic markers, the pro-inflammatory cytokines of serum IL-17A, IL-6, IL-1β, IFN-γ and TNF-α were analyzed. Serum IL-6 levels (Fig. 3A) were significantly reduced in the HF + PFD group (145.8 ± 15.0 ng/ml) compared to the HF group (211 ± 30 ng/ml). The serum levels of IL-1β (Fig. 3B) significantly shows the reduction in the HF + PFD group (69.3 ± 13 ng/ml) compared to the HF group (177.4 ± 20.6 ng/ml). IFN-γ is shown in Fig. 3C and was significantly lower in the PFD-LP group (18.2 ± 8 ng/ml) compared to the HF group (221.3 ± 50 ng/ml). Similarly, TNF-α (Fig. 3D) shows a significant reduction in serum levels in the HF + PFD group (32.9 ± 21.1 ng/ml) compared to the HF group (254.6 ± 70 ng/ml). Worthy of mention, the level of IL-17A (Fig. 3F), showed a dramatic reduction in mice treated with PFD-LP (271.1 ± 149.6) compared to the HF group, which showed a significant increase in serum levels of IL-17A (1983.5 ± 400 ng/ml). All the cytokines analyzed in the HF group showed a highly significant increase compared to the control group. Finally, it was observed that the serum level of the anti-inflammatory cytokine IL-10 (Fig. 3E) was significantly higher in the HF + PFD group (37.8 ± 10.4 ng/ml) compared to the HF group (15.4 ± 9.1 ng/ml).

### EXPRESSION OF PROFIBROGENIC AND PROINFLAMATORY MARKER GENES

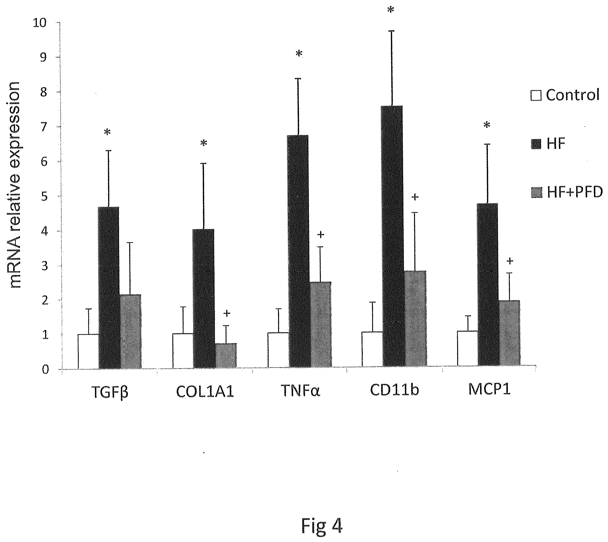

The level of liver messenger RNA showed a decrease in the expression of TGF-β1, and a significant down-regulation of COL1A1 and TNF-α in the PFD-LP + HF group. In addition, a significant reduction in the expression of genes CD11b and MCP1 was observed in comparison with the HF group (Fig. 4).

PFD-LP MODULATES THE MEDIATORS OF THE METABOLISM OF THE HEPATIC FATS.

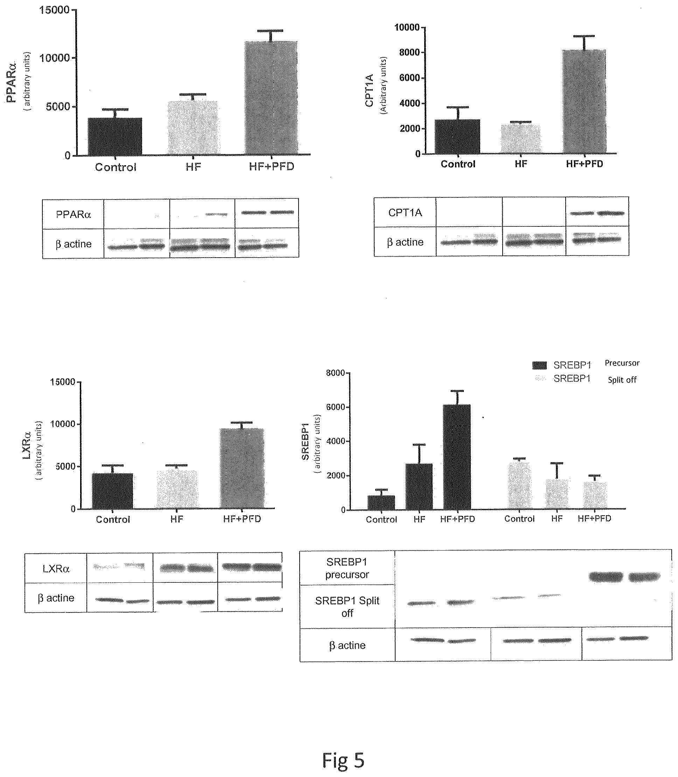

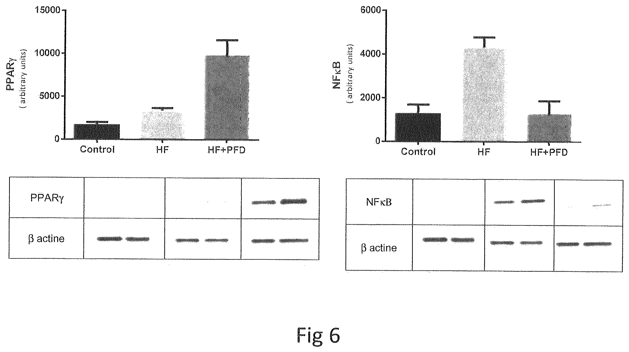

To analyze the effect of PFD on the modulation of fat metabolism mediators in NAFLD/NASH, the key metabolic transcription factors LXR and PPARalpha were evaluated in liver tissue. As shown in Fig. 5, the protein levels of LXR (8082 ± 847) and PPARalpha (11506 ± 1167) increased significantly in the PFD + HF group compared to the control (2,634 ± 1,042 and 3,890 ± 1,130, respectively) and the HF group (3375 ± 898 and 7308 ± 1117 respectively). To understand better the effect of PFD-LP on key proteins such as SREBP1 and CPT1A that are targeted by LXR and PPAR respectively, they were also evaluated. As shown in Fig. 5, PFD-LP induced an increase in the precursor protein SREBP1 (6057 ± 847) compared to the control (785 ± 396) and the HF group (2,622 ± 1,161). However, SREBP1 in its cleaved form (active form) showed a tendency to decrease, but no significant differences were found between the three groups (4567 ± 1620), HF (1776 ± 893) and PFD + HF (1638 ± 303). In addition, a significant increase was observed in CPT1A expression (enzyme responsible for internalizing hepatic fats in the mitochondria to be "burned") in the PFD + HF group (9303 ± 809), compared to the control (4303 ± 820) and the HF group (6.172 ± 1.356). Finally, Fig. 6 shows us that key transcriptional factors in the regulation of the inflammatory process in the liver such as PPARgamma and NFkB are specifically modulated in a way that results in the decrease of hepatic inflammation.

### BIBLIOGRAPHY

1. Marra F, Lotersztajn S. Pathophysiology of NASH: perspectives for a targeted treatment. Curr Pharm Des 2013;19(29):5250-5269.
2. Ferramosca A, Vincenzo Zara. Modulation of hepatic steatosis by dietary fatty acids. World J Gastroenterol 2014;20(7):1746-1755
3. Paschos P, Paletas K. Non alcoholic fatty liver disease and metabolic syndrome. Hippokratia 2009;13(1):9-19.
4. Van Rooyen DM, Larter CZ, Haigh WG, Yeh MM, Ioannou G, Kuver R, et. al. Hepatic free cholesterol accumulates in obese, diabetic mice and causes nonalcoholic steatohepatitis. Gastroenterology 2011;141:1393-403.
5. Kang H, Greenson JK, Omo JT, Guillot C, Peterman D, Anderson L, et. al. Metabolic syndrome is associated with greater histologic severity, higher carbohydrate, and lower fat diet in patients with NAFLD. Am J Gastroenterol 2006;101:2247-53.
6. Lim JS, Mietus-Snyder M, Valente A, Schwarz JM, Lustig RH. The role of fructose in the pathogenesis of NAFLD and the metabolic syndrome. Nat Rev Gastroenterol Hepatol 2010;7:251-64.
7. Harley IT, Stankiewicz TE, Giles DA, Softic S, Flick LM, Cappelletti M, et al. IL-17 signaling accelerates the progression of nonalcoholic fatty liver disease in mice. Hepatology. 2014;59(5):1830-9.
8. Zúñiga LA, Shen W-J, Joyce-Shaikh B, Pyatnova EA, Richards AG, Thom C, et al. IL-17 Regulates Adipogenesis, Glucose Homeostasis, and Obesity. J Immunol. 2010 Dec 1;185(11):6947-59. Lafdil F, Miller AM, Ki SH, Gao B. Th17 cells and their associated cytokines in liver diseases. Cell Mol Immunol 2010;7(4):250-254.
9. Weaver CT, Hatton RD. Interplay between the TH17 and Treg cell lineages: a co-evolutionary perspective. Nat Rev Immunol 2009;9:883-9.
10. Korn T, Bettelli E, Oukka M, Kuchroo VK. IL-17 and Th17 Cells. Annu Rev Immunol 2009;27:485-517.
11. Tang Y, Bian Z, Zhao L, Liu Y, Liang S, Wang Q, et. al. Interleukin-17 exacerbates hepatic steatosis and inflammation in non-alcoholic fatty liver disease. Clin Exp Immunol 2011;166(2):281-90.
12. Wang Y-X. PPARs: diverse regulators in energy metabolism and metabolic diseases. Cell Res. 2010;20(2):124-37.
13. Yan Xing, Tingting Zhao, Xiaoyan Gao, Yuzhang Wu. Liver X receptor α is essential for the capillarization of liver sinusoidal endothelial cells in liver injury.
14. Yoshikawa T, Ide T, Shimano H, Yahagi N, Amemiya-Kudo M, Matsuzaka T, et al. Cross-Talk between Peroxisome Proliferator-Activated Receptor (PPAR) α and Liver X Receptor (LXR) in Nutritional Regulation of Fatty Acid Metabolism. I. PPARs Suppress Sterol Regulatory Element Binding Protein-1c Promoter through Inhibition of LXR Signaling. Mol Endocrinol. 2003;17(7):1240-54.
15. Cui G, Qin X, Wu L, Zhang Y, Sheng X, Yu Q, Sheng H, Xi B, Zhang JZ, Zang YQ. Liver X receptor (LXR) mediates negative regulation of mouse and human Th17 differentiation. J Clin Invest. 2011;121(2):658-670.
16. Ducheix S, Montagner A, Polizzi A, Lasserre F, Marmugi A, Bertrand-Michel J, et al. Essential fatty acids deficiency promotes lipogenic gene expression and hepatic steatosis through the liver X receptor. J Hepatol. 2013;58(5):984-92.

## Claims

1. A pharmaceutical composition in the form of an extended-release tablet comprising 100 mg, 200 mg, 300 mg, 400 mg, or 600 mg of pirfenidone for use in the treatment of non-alcoholic steatohepatitis (NASH).

2. The pharmaceutical composition for use according to claim 1, wherein the use decreases serum cholesterol and triglycerides.

3. The pharmaceutical composition for use according to claim 1 or 2, wherein the use decreases the content of hepatic fat accumulation.

4. The pharmaceutical composition for use according to any one of claims 1 to 3, wherein the use induces a decrease in the expression of the nuclear factor kappa-light-chain-enhancer of activated B cells (NFkB).

5. The pharmaceutical composition for use according to claim 3, wherein the hepatic fat accumulation is microvesicular steatosis.

6. The pharmaceutical composition for use according to claim 3, wherein the hepatic fat accumulation is macrovesicular steatosis.

7. The pharmaceutical composition for use according to any one of claims 1 to 6, wherein the extended-release tablet comprises 100 mg of pirfenidone.

8. The pharmaceutical composition for use according to any one of claims 1 to 6, wherein the extended-release tablet comprises 200 mg of pirfenidone.

9. The pharmaceutical composition for use according to any one of claims 1 to 6, wherein the extended-release tablet comprises 300 mg of pirfenidone.

10. The pharmaceutical composition for use according to any one of claims 1 to 6, wherein the extended-release tablet comprises 400 mg of pirfenidone.

11. The pharmaceutical composition for use according to any one of claims 1 to 6, wherein the extended-release tablet comprises 600 mg of pirfenidone.

## Patentansprüche

1. Pharmazeutische Zusammensetzung in Form einer Retardtablette, umfassend 100 mg, 200 mg, 300 mg, 400 mg, oder 600 mg Pirfenidon, zur Verwendung bei der Behandlung von nichtalkoholischer Steatohepatitis (NASH).

2. Pharmazeutische Zusammensetzung zur Verwendung nach Anspruch 1, wobei durch die Verwendung Serumcholesterin und Triglyceride vermindert werden.

3. Pharmazeutische Zusammensetzung zur Verwendung nach Anspruch 1 oder 2, wobei durch die Verwendung der Gehalt an Leberfettakkumulation vermindert wird.

4. Pharmazeutische Zusammensetzung zur Verwendung nach einem der Ansprüche 1 bis 3, wobei durch die Verwendung eine Abnahme der Expression von NF-xB (Nuclear Factor kappa-light-chain-enhancer of activated B cells) induziert wird.

5. Pharmazeutische Zusammensetzung zur Verwendung nach Anspruch 3, wobei es sich bei der Leberfettakkumulation um mikrovesikuläre Steatose handelt.

6. Pharmazeutische Zusammensetzung zur Verwendung nach Anspruch 3, wobei es sich bei der Leberfettakkumulation um makrovesikuläre Steatose handelt.

7. Pharmazeutische Zusammensetzung zur Verwendung nach einem der Ansprüche 1 bis 6, wobei die Retardtablette 100 mg Pirfenidon umfasst.

8. Pharmazeutische Zusammensetzung zur Verwendung nach einem der Ansprüche 1 bis 6, wobei die Retardtablette 200 mg Pirfenidon umfasst.

9. Pharmazeutische Zusammensetzung zur Verwendung nach einem der Ansprüche 1 bis 6, wobei die Retardtablette 300 mg Pirfenidon umfasst.

10. Pharmazeutische Zusammensetzung zur Verwendung nach einem der Ansprüche 1 bis 6, wobei die Retardtablette 400 mg Pirfenidon umfasst.

11. Pharmazeutische Zusammensetzung zur Verwendung nach einem der Ansprüche 1 bis 6, wobei die Retardtablette 600 mg Pirfenidon umfasst.

## Revendications

1. Composition pharmaceutique sous la forme d'un comprimé à libération prolongée comprenant 100 mg, 200 mg, 300 mg, 400 mg, ou 600 mg de pirfénidone pour une utilisation dans le traitement de la stéatohépatite non alcoolique (SHNA).

2. Composition pharmaceutique pour une utilisation selon la revendication 1, où l'utilisation diminue le cholestérol et les triglycérides sériques.

3. Composition pharmaceutique pour une utilisation selon la revendication 1 ou 2, où l'utilisation diminue le taux d'accumulation de graisse hépatique.

4. Composition pharmaceutique pour une utilisation selon l'une quelconque des revendications 1 à 3, où l'utilisation induit une diminution de l'expression du facteur nucléaire améliorateur de chaîne légère kappa de cellules B activées (NFκB).

5. Composition pharmaceutique pour une utilisation selon la revendication 3, où l'accumulation de graisse hépatique est une stéatose microvésiculaire.

6. Composition pharmaceutique pour une utilisation selon la revendication 3, où l'accumulation de graisse hépatique est une stéatose macrovésiculaire.

7. Composition pharmaceutique pour une utilisation selon l'une quelconque des revendications 1 à 6, où le comprimé à libération prolongée comprend 100 mg de pirfénidone.

8. Composition pharmaceutique pour une utilisation selon l'une quelconque des revendications 1 à 6, où le comprimé à libération prolongée comprend 200 mg de pirfénidone.

9. Composition pharmaceutique pour une utilisation selon l'une quelconque des revendications 1 à 6, où le comprimé à libération prolongée comprend 300 mg de pirfénidone.

10. Composition pharmaceutique pour une utilisation selon l'une quelconque des revendications 1 à 6, où le comprimé à libération prolongée comprend 400 mg de pirfénidone.

11. Composition pharmaceutique pour une utilisation selon l'une quelconque des revendications 1 à 6, où le comprimé à libération prolongée comprend 600 mg de pirfénidone.
